Europäisches Patentamt

(19) European Patent Office

Office européen des brevets

(11) Numéro de publication: **0 184 571**
**A1**

## (12) DEMANDE DE BREVET EUROPEEN

(21) Numéro de dépôt: **85870163.4**

(22) Date de dépôt: **26.11.85**

(51) Int. Cl.⁴: **B 01 L 3/14**
**G 01 N 33/48**

(30) Priorité: **28.11.84 BE 214073**

(43) Date de publication de la demande:
**11.06.86 Bulletin 86/24**

(84) Etats contractants désignés:
**AT BE CH DE FR GB IT LI LU NL SE**

(71) Demandeur: **Etablissements Pasture et Cie.**
**Avenue Rogier 158**
**B-1050 Bruxelles(BE)**

(72) Inventeur: **Delmotte, Yves**
**rue de la Fontaine 36**
**B-7340 Tertre(BE)**

(74) Mandataire: **Callewaert, Jean et al,**
**Bureau Gevers S.A. rue de Livourne, 7 - Bte 1**
**B-1050 Bruxelles(BE)**

(54) Colonne d'extraction et/ou de filtration.

㊼ Colonne d'extraction et/ou de filtration constituée de plusieurs parties démontables, une première partie (2) présentant un logement (5) destiné à contenir une matière poreuse pouvant être traversée par un liquide introduit dans un réservoir (6) situé au-dessus de ce logement (5), une deuxième partie (3) présentant au moins un réservoir de collection (7) pour le liquide ayant traversé la matière poreuse et pouvant s'adapter en dessous de ladite première partie. Lorsque les deux parties (2) et (3) sont adaptées l'une sur l'autre, le logement (5) se situe à une distance suffisante du fond du réservoir (7) pourque le liquide recueilli dans ce dernier ne mouille pas la matière poreuse contenue dans le logement.

FIG. 2

EP 0 184 571 A1

Croydon Printing Company Ltd.

La présente invention est relative à une colonne d'extraction et/ou de filtration constituée de plusieurs parties démontables pouvant s'adapter l'une sur l'autre suivant l'axe de la colonne, une première partie présentant un logement destiné à contenir une matière poreuse, telle qu'une matière filtrante et/ou une résine échangeuse d'ions, d'adsorption de partition, polaire et non-polaire, pouvant être traversée par un liquide introduit dans un réservoir situé au-dessus de ce logement, une deuxième partie présentant au moins un réservoir de collection pour le liquide ayant traversé la matière poreuse et pouvant s'adapter en dessous de ladite première partie.

Il s'agit plus particulièrement de colonnes utilisées pour la réalisation de tests analytiques, préparatifs et d'enrichissement d'échantillons de différentes liquides généralement de nature organique, tels que du sang, de l'urine, etc...

Les colonnes d'extraction et de filtration du type précité, utilisé jusqu'à présent, soit ne peuvent pas être utilisées dans une centrifugeuse, soit nécessitent une centrifugeuse particulière d'une construction relativement compliquée et donc relativement coûteuse.

Le but de la présente invention consiste essentiellement à proposer une colonne d'extraction et/ou de filtration pouvant être placée dans n'importe quel type de centrifugeuse connue, notamment pour le traitement de liquides contenus dans des éprouvettes.

A cet effet, suivant l'invention, les parties précitées sont assemblées l'une à l'autre de manière démontable, le logement, destiné à contenir la matière poreuse, se situant, en position assemblée, à une distance suffisante du fond du réservoir de collection pour "outermost" bearing of the gimbal-ring type or universal suspension, it is not seen how this further problem can be readily solved.

The solution afforded by the universal or gimbal-ring type suspension is of principle interest, however, with respect to the possibilities of achieving rapid motion. This applies particularly to constructions which incorporate articulated arms and the like, since the fact that the drive motors must be placed adjacent to or preferably in a stationary mounting part presents a further problem.

An object of the present invention is to provide a rapidly operating and accurate industrial robot constructed on the basis of the known gimbal-ring type suspension. In this respect the use of heavy motors and like devices shall not render the arrangement sluggish when mounted in the proximity of the principal pivot points of the system. The invention is thus based on the view that, contrary to the proposals set forth in the aforesaid Japanese application, it is unnecessary to mount all motors on the stationary mounting of the robot, but that the motors shall instead be mounted in the vicinity of points at which the principal pivot or swinging axes intersect, therewith obviating, in accordance with the invention, the requirement that they shall be immovable.

More specifically, these and other advantages together with further objects of the invention are achieved with an industrial robot of the kind mentioned in the introduction having the characteristic features set forth in the characterizing clause of Claim 1. The gimbal-ring type suspended part suitably has the form of a box-like structure or a tube of preferably rectangular cross-section, in which guide means are arranged. The platform, which thus carries three motors with associated ro... ... in relation to the pivot centre of the cardanic suspension

Les figures 7 à 9 concernent des vues en élévation et en coupe longitudinale d'accessoires suivant différentes formes de réalisation pouvant être placées dans la colonne suivant les figures 1, 2 et 4 à 6.

La figure 10 est une coupe longitudinale d'une troisième forme de réalisation de l'invention, à l'état démonté.

Dans les différentes figures, les mêmes chiffres de référence concernent des éléments identiques ou analogues.

La colonne d'extraction et/ou de filtration qui fait l'objet de la présente invention porte la référence 1 sur les figures annexées et est en particulier destinée pour des tests analytiques de différents types de liquides, tels que des échantillons de solutions organiques, plus particulièrement de sang, d'urine etc...

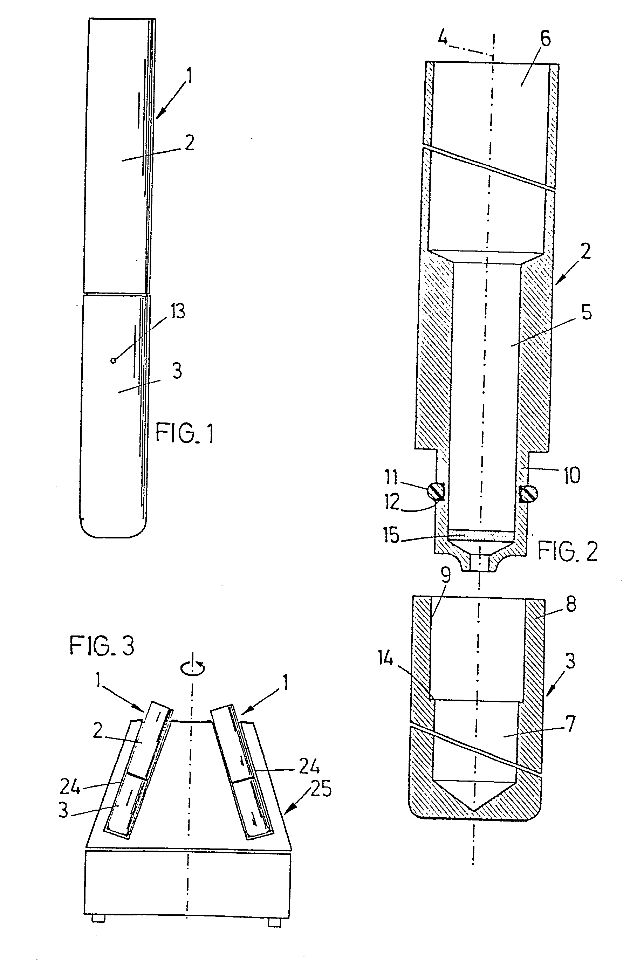

Les figures 1 et 2 montrent une première forme de la réalisation d'une telle colonne. Celle-ci est constituée de deux parties démontables 2 et 3 pouvant s'adapter l'une sur l'autre suivant l'axe 4 de la colonne.

La première partie 2 présente un logement 5 destiné à contenir une matière poreuse non représentée aux figures, telle qu'une matière filtrante et/ou une résine échangeuse d'ions, d'adsorption de partition, polaire et non-polaire, pouvant être traversée par un liquide introduit dans un réservoir 6 situé au-dessus du logement 5.

La deuxième partie 3 comprend un réservoir de collection 7 pour le liquide ayant traversé la matière poreuse et peut s'adapter en-dessous de la partie 2.

Suivant l'invention, lorsque les deux parties 2 et 3 de la colonne 1 sont adaptées l'une sur l'autre, comme représentées à la figure 1, le logement 5, destiné à contenir la matière poreuse, se situe à une distance suffisante du fond du réservoir de collection 7 pourque le liquide recueilli dans ce dernier ne mouille pas cette matière poreuse.

Ceci revient donc au fait que le volume libre de ce réservoir 7 est au moins égal au volume du liquide pouvant traverser en une même opération la matière poreuse contenue dans le logement 5.

Une autre caractéristique essentielle liée à celle du réservoir 7 est que des moyens sont prévus pour empêcher que les deux parties démontables 2 et 3 puissent se détacher sous le simple effet de la pesanteur, p.e. lorsqu'on soulève la colonne en la saisissant uniquement par la partie supérieure 2. Ceci peut être très important lorsque la colonne doit être posée dans une centrifugeuse classique à alvéoles ou emplacements présentant un fond fermé.

Le logement 5 se situe de préférence essentiellement au dessus du réservoir de collection 7 lorsque les deux parties 2 et 3 sont assemblées, de manière à pouvoir collecter ainsi un volume aussi important que possible dans le réservoir 7.

Le réservoir 7 est surmonté d'un manchon 8 présentant une surface cylindrique intérieure 9 évasée par rapport au réservoir 7 et pouvant glisser sur un embout 10 s'étendant en dessous de la partie 2 de la colonne. Un joint d'étanchéité, formé par un "O-ring" 11, est maintenu dans une rainure circulaire 12 pratiquée dans cet embout 10 de manière à permettre d'adapter le manchon 8 d'une manière immobile et facilement démontable sur celui-ci. Ce joint 11 constitue donc une forme de réalisation particulière des moyens précités.

Un orifice 13 d'un diamètre relativement réduit, de l'ordre de 1 mm est prévu à proximité du bord supérieur 14 dans la paroi latérale du réservoir de collection 7 pour permettre l'évacuation de l'air contenu dans ce dernier lorsque du liquide y est recueilli.

Le fond du logement 5 peut présenter un support perméable 15 sur lequel repose alors la matière poreuse.

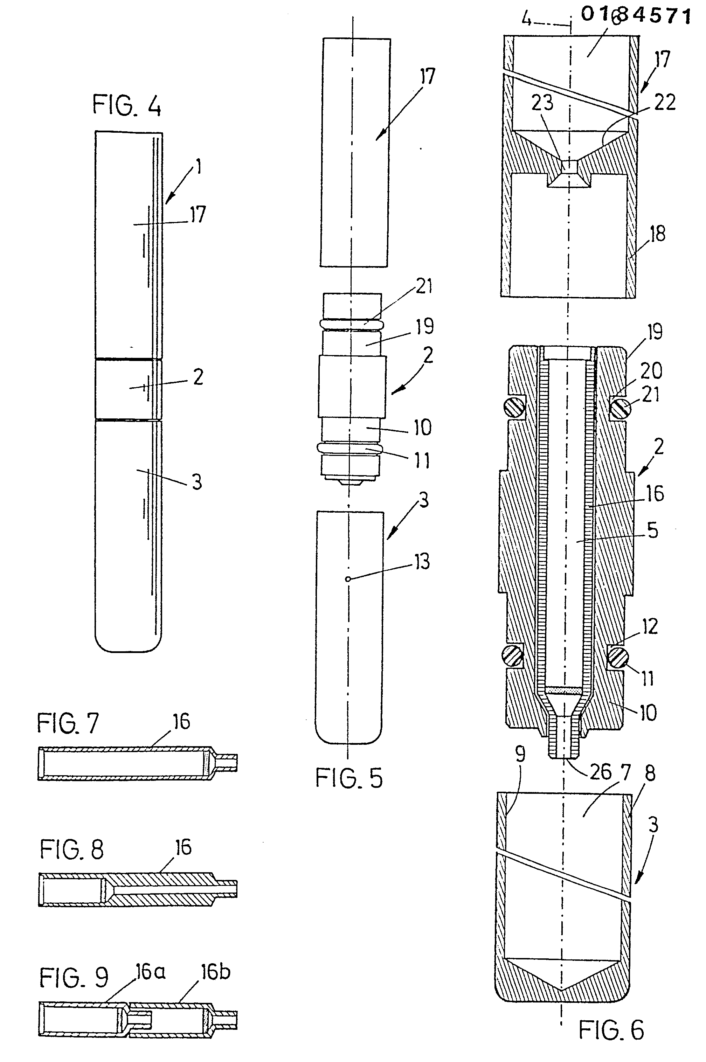

Cette dernière peut être introduite initialement sous forme d'une suspension ou être contenue, à l'état sec, dans au moins une cartouche 16 (voir figures 7 à 9) pouvant être placée dans le logement 5.

Le cas échéant, cette matière poreuse peut être enfermée dans plusieurs cartouches séparées superposées, communiquant en série l'une avec l'autre. A cet égard, la figure 9 montre deux cartouches 16a et 16b s'emboîtant partiellement l'une dans l'autre.

Les figures 4 à 6 montrent une deuxième forme de réalisation de la colonne d'extraction et/ou de filtration suivant l'invention.

Cette colonne se distingue essentiellement par rapport à celle montrée aux figures 1 et 2 par le fait que le réservoir 6, pour le liquide à faire passer à travers la matière poreuse, est agencé dans une troisième partie démontable 17 s'adaptant par l'intermédiaire d'un manchon 18 sur un embout 19 prévu du côté supérieur de la première partie 2 de la colonne.

Comme pour l'embout 10, situé du côté opposé, l'embout 19 présente une rainure circulaire 20 dans laquelle est logée un O-ring 21 permettant de placer d'une manière étanche le manchon 18 sur cet embout 19.

Dans cette deuxième forme de réalisation, la partie 2 de la colonne ne comprend plus que le logement 5 qui s'étend sur toute la hauteur de celle-ci.

Le fond 22 du réservoir 6 prévu dans la partie 17 de la colonne 1 est conique et présente en son centre une ouverture de sortie 23 pour le liquide, débouchant au dessus du logement 5 ménagé dans la deuxième partie 2.

Le manchon 8 de la deuxième partie 3 de la colonne présente une surface intérieure s'étendant dans le prolongement de celle du réservoir 7, donc contrairement à ce qui est le cas pour la forme de réalisation suivant la figure 2. Cette surface peut éventuellement être légèrement conique pour faciliter la mise en place du manchon 8 sur l'embout 10, ce dernier étant dans ce cas également légèrement conique.

Au moins la deuxième partie 3, formant donc la partie inférieure de la colonne 1, présente une forme extérieure compatible avec des compartements 24 d'une centrifugeuse classique 25 (voir figure 3).

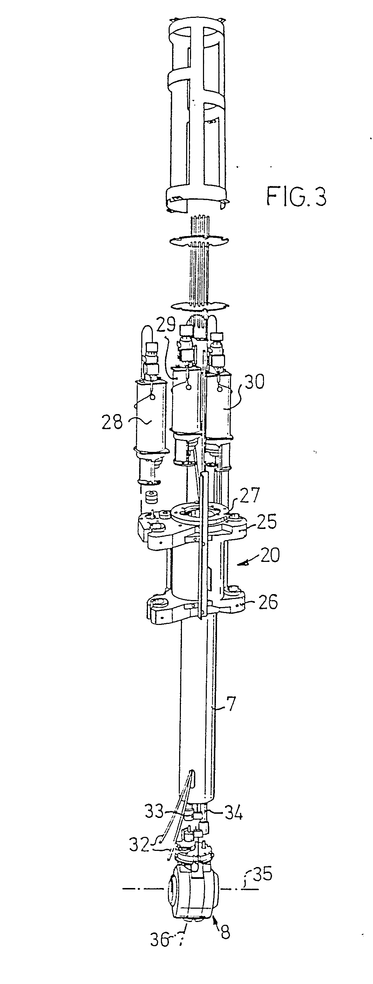

La troisième forme de réalisation de la colonne, suivant l'invention, représentée à la figure 10, se distingue essentiellement par rapport à la deuxième forme de réalisation telle que montrée aux figures 4 à 6, par le fait que la partie 2, contenant la matière poreuse, est constituée en son entier par une cartouche échangeable 16 intercalée de manière démontable entre les parties 3 et 17. Cette partie 2 et la cartouche 16 sont ainsi constituées par la même pièce. Ceci n'est donc pas le cas pour la deuxième forme de réalisation où la partie 2 présente un logement 5 dans lequel la cartouche 16 est glissée.

Par ailleurs, dans cette troisième forme de réalisation l'embout 10 présente une conicité quelque peu plus prononcée que dans cette deuxième forme de réalisation. Cet embout 10 est engagé au moins partiellement dans le manchon 8 du réservoir 7 de la partie 3 de la colonne qui présente une paroi intérieure à conicité correspondante de manière à permettre de maintenir l'embout 10 dans le manchon 9 par simple coinçage ou frottement, et ceci sans qu'un joint d'étanchéité p.e. sous forme d'un O-ring, soit nécessaire.

Une liaison analogue est prévue entre la partie démontable 17 et la partie 2 ou la cartouche 16.

Cette liaison comprend un embout conique 18 s'engageant dans l'embout 19 de la cartouche 16 présentant une paroi intérieure 27 présentant une conicité correspondante.

Dans cette forme de réalisation les moyens pour empêcher que les parties démontables puissent se détacher sous le simple effet de la pesanteur sont donc formés par ces conicités.

Les trois parties 2, 3 et 17 de la colonne sont de préférence réalisées en une matière plastique relativement souple inerte aux solvants, telle que polypropylène, de qualité médical, de sorte que, grâce à l'élasticité de cette matière, ces parties peuvent être assemblées de manière étanche.

Dans cette dernière forme de réalisation la matière poreuse a été représentée et a été désignée par la référence 28. Elle peut occuper tout le volume du logement 5 ou seulement une partie de celui-ci, comme dans la figure 10, suivant l'application envisagée.

Une pastille poreuse 30 peut être placée dans le logement 5, au-dessus de la matière poreuse 28 pour maintenir cette dernière en place et éventuellement pour la comprimer quelque peu.

Les parois extérieures 29 dans la partie 2 peuvent être nervurées pour faciliter la préhension. Par ailleurs, des rainures axiales, non représentées, peuvent être prévues dans la paroi extérieure de l'embout 10, en remplacement de l'orifice 13 dans la forme de réalisation de la figure 1, pour permettre l'évacuation de l'air lorsque du liquide est recueilli dans le réservoir de la partie 3 adaptée sur la partie 2.

Avantageusement, comme montré aux différentes figures, les parties démontables précitées adaptées l'une sur l'autre forment un ensemble ayant l'allure d'une éprouvette pouvant être placé dans un compartiment 24 d'une centrifugeuse 25.

Dans certains cas, les compartiments de la centrifugeuse pourraient être des supports de dimensions relativement réduites et être formés par exemple par des anneaux dans lesquels la colonne 1 est suspendue par sa partie inférieure 3. Il faudrait alors que cette partie présente de préférence un rebord de retenu avec lequel la colonne peut être posée dans cet anneau. Un tel rebord n'a, toutefois, pas été représenté aux figures.

Grâce à cette possibilité de pouvoir faire usage d'une centrifugeuse pour passer le liquide à traiter à travers la matière poreuse, le diamètre de l'ouverture de sortie 26 du logement 5 ou des cartouches 16 peut être relativement réduite, p.e. de l'ordre de 1 mm. Dans ces conditions, si on fait usage de colonnes contenant déjà de la résine en suspension dans un liquide, il n'est pas nécessaire de prévoir des moyens de fermeture de cette ouverture pour le stockage de ces colonnes.

La façon dont la colonne, suivant l'invention, est utilisée varie essentiellement en fonction de la nature des échantillons de liquide à traiter et des tests à effectuer.

Si par exemple une molécule ou un groupe de molécules doit être dosé dans un échantillon déterminé, on fait usage, comme matière poreuse à introduire dans le logement 5 de la colonne, d'une résine qui permet de capter sélectivement cette molécule ou ce groupe de molécules.

Cette résine peut être introduite dans la colonne à l'état sec, sous forme de cartouches ou non, ou comme suspension

Dans ce dernier cas, une première opération consistera à séparer la phase liquide de la résine en soumettant la colonne, avec ses parties démontables adaptées l'une sur l'autre, à une centrifugation, comme montrée par exemple à la figure 3.

La phase liquide est recueilli dans le réservoir 7 et peut donc être éliminée.

La résine est retenue, pratiquement exempt de liquide, dans le logement 5 grâce à la présence du support perméable 15. De plus, par suite de l'action de la force centrifuge un tassement convenable de la résine a été obtenu et ce qui permet donc d'éviter la formation de passages préférentiels pour l'échantillon à analyser.

Cette première opération peut être évitée en faisant usage de cartouches 16 ou de résines à l'état sec.

L'opération suivante consiste à verser l'échantillon dans le réservoir 6 de la colonne, au-dessus du logement 5 contenant la résine et après avoir fixé à nouveau la partie 3 sur l'embout 10 de la partie 2, à soumettre l'ensemble à une nouvelle centrifugation selon des normes bien définies pour ce qui concerne les différents paramètres, tels que nombres de tours, durée de rotation etc...

Sous l'action de la force centrifuge, l'échantillon est forcé au travers du lit de résine contenu dans le logement 5. Les molécules à extraire de l'échantillon sont alors fixées de manière sélective sur la résine.

Selon le même principe, on procède ensuite à un lavage avec un volume $V_1$ bien déterminé et une élution au moyen d'une solution appropriée également d'un volume déterminé $V_2$.

Par le lavage, les substances interférentes sont éliminées de la résine et, au moyen de la solution d'élution, les molécules à doser ainsi que leur standard interne sont alors décrochés de la résine et recueillis avec cette solution dans le réservoir 7 de la partie inférieure 3 de la colonne.

Ensuite, les molécules récoltées dans la solution d'élution peuvent être dosées quantitativement, par exemple par la méthode chromatographique suivant une manière connue en soi.

Pour certaines analyses il pourrait être utile de faire usage d'au moins deux types différents de résine présentant des affinités d'adsorption pour des molécules différentes.

On peut ainsi faire usage d'un mélange de ces résines soit dans une suspension, soit dans une même cartouche, soit encore dans des cartouches différentes montées en série dans le logement 5, comme montré à la figure 9.

Les molécules adsorbées sur chacunes des résines peuvent alors être séparées par l'utilisation successive de solutions d'élution suffisamment sélectives.

Grâce à la possibilité d'éliminer par ladite centrifugation pratiquement la totalité de la phase liquide après chaque opération, il est possible d'éviter toute contamination par "carry-over". Ceci est surtout important si on veut doser dans un même échantillon différents types de molécules. Il est, en effet, possible d'éviter que des molécules fixées sur un type de résine déterminé puissent être entraînées par une solution d'élution destinée à décrocher un autre type de molécule adsorbé par une autre résine contenue également dans le logement 5.

Par ailleurs, parsuite de la possibilité de contrôler rigoureusement les différentes paramètres de la centrifugation les tests analytiques sont parfaitement reproductibles.

Il existe également la possibilité de conditionner les colonnes en fonction du type de resine choisie, de la vitesse de rotation de la centrifugeuse pour obtenir un tassement approprié.

Un autre avantage encore est le rendement de récupération très élevé des molécules à doser.

L'intérêt de la colonne d'extraction et/ou de filtration suivant l'invention est illustré d'une manière plus concrète par l'exemple donné ci-après.

<u>Exemple</u>

<u>Dosage simultanée du pregnandiol et de l'estriol.</u>

1)Prise d'essai - hydrolyse
2ml d'urine filtrée ont été mélangés avec 0,2 ml d'hélicase, 4 ml de tampon pH 4,8 et 100 µl de standard interne (Epicoprostanol)
Le mélange a ensuite été placé pendant 1 heure au bain-marie à 56°C
2) Extraction

L'échantillon ainsi préparé a alors été soumis à une extraction dans une colonne du type montré aux figures 1 et 2.

A cet effet, cette colonne a été remplie avec une suspension de 3 ml de résine échangeuse d'anions fortement basique d'une grannulométrie de 50 à 100 mesh du type AG -1-X4 de la société Bio-Rad.

La phase liquide a été éliminée et la résine restante a été tassée dans la colonne en soumettant cette dernière à une centrifugation pendant environ 30 secondes à 2000 tours par minute. Le filtrat a été recueilli dans le réservoir 7 et a été évacué.

La résine a ensuite été lavée avec 4 ml de tampon pH 4,1 et soumise à une nouvelle centrifugation à 2000 tours par minute.

L'eau de lavage recueillie a été à son tour éliminé.

Après ce lavage, de la résine, l'échantillon d'urine hydrolysé a été versé dans le réservoir supérieur 6 de la colonne qui a alors été soumise à une troisième centrifugation toujours à 2000 tours par minute.

Dans une quatrième étape, la résine a été traversée par une solution d'élution, formée de 5 ml de méthanol, qui a été introduite dans le réservoir 6 de la colonne en soumettant à nouveau cette dernière à une centrifugation à 2000 tours par minute pendant 30 à 40 secondes en fonction de la nature du lit de résine.

L'éluat obtenu dans le réservoir 7 a été transvasé dans un tube en verre et a été évaporé sous atmosphère d'azote.

3) Analyse

100 µl d'un agent de silylation formé de BSTFA/TMCS 4 : 1 V/V a été versé dans ce tube qui a été bouché hermétiquement, porté pendant 20 minutes à 65 à 70°C et refroidi à température ambiante.

Le dosage du pregnandiol et de l'estriol a alors été réalisé par chromatographie gazeuse dans les conditions suivantes :

Sur colonne OV-1 : Température du four isotherme à 250°

Attenuation 32

Range 10

Temps de retention :             Pregnandiol 10 min

                                 Estriol 12 min Epicoprostanol 14 min

Cet exemple type illustre le principe et les possibilités d'utilisation de la cartouche.

De nombreuses applications sont disponibles comme le dosage de l'acide vanillimandelique (VMA), des Catecholamines, des métanephrines sur matrice urinaire, dosages sériques concernant la toxicologie etc...

Il est bien entendu que l'invention n'est pas limitée aux formes de réalisation décrites ci-dessus de la colonne d'extraction et/ou de filtration mais que bien des variantes peuvent être envisagées sans sortir du cadre de l'invention, notamment en ce qui concerne la forme et les dimensions des différentes parties constituantes de la colonne.

C'est ainsi que p.e. les moyens permettant d'empêcher le désassemblage des parties démontables sous l'effet de la pesanteur peuvent être variés. Ainsi, un embout 10 et machon 8 d'allure conique peuvent également être prévus aux première et deuxième formes de réalisation décrites ci-dessus en remplacement des joints d'étanchéité 11.

- 12 -                                    0184571

## REVENDICATIONS

1. Colonne d'extraction et/ou de filtration (1) constituée de plusieurs parties démontables pouvant s'adapter l'une sur l'autre suivant l'axe de la colonne, une première partie (2) présentant un logement (5) destiné à contenir une matière poreuse 28, telle qu'une matière filtrante et/ou une résine échangeuse d'ions, d'adsorption ou de partition, polaire et non-polaire, pouvant être traversée par un liquide introduit dans un réservoir (6) situé au-dessus de ce logement, une deuxième partie (3) présentant au moins un réservoir de collection (7) pour le liquide ayant traversé la matière poreuse et pouvant s'adapter en dessous de ladite première partie (2), cette colonne (1) étant caractérisée en ce que les parties précitées (2) et (3) ont assemblées l'une à l'autre de manière démontable, le logement (5), destiné à contenir la matière poreuse (28), se situant, en position assemblée des parties, à une distance suffisante du fond du réservoir de collection (7) pourque le liquide ayant traversé la matière poreuse et recueilli dans ce dernier ne mouille pas la matière poreuse contenue dans le logement (5), des moyens (8, 9, 10, 11, 18, 19, 27) étant prévus permettant d'éviter le désassemblage desdites parties sous l'effet de la pesanteur.

2. Colonne suivant la revendication 1, caractérisée en ce que le logement précité (5) se situe essentiellement au dessus du réservoir de collection (7) lorsque les deux parties (2) et (3) précités sont adaptées l'une sur l'autre.

3. Colonne suivant l'une ou l'autre des revendications 1 et 2, caractérisée en ce que les deux parties précitées (2) et (3) s'adaptent l'une sur l'autre par l'intermédiaire d'un joint d'étanchéité (11), un orifice (13) étant prévu à proximité du bord supérieur de la paroi latérale du réservoir de collection (7) pour permettre l'évacuation de l'air contenu dans ce dernier lorsque du liquide y est recueilli.

4. Colonne suivant l'une quelconque des revendications 1 à 3, caractérisée en ce que la matière poreuse est contenue dans au moins une cartouche (16) pouvant être placée dans le logement précité (5).

5. Colonne suivant la revendication 4, caractérisée en ce que la matière poreuse est contenue dans au moins deux cartouches (16a, 16b) séparées et superposées communiquant en série l'une avec l'autre.

6. Colonne suivant l'une quelconque des revendications 1 à 5, caractérisée en ce que le réservoir (6) pour du liquide à faire passer à travers la matière poreuse est agencé dans une troisième partie démontable (17) s'adaptant au-dessus de la première partie précitée (2) de la colonne (1).

7. Colonne suivant la revendication 6, caractérisée en ce que cette troisième partie (17) est pourvue, à son extrémité inférieure, d'un manchon (18) s'adaptant par l'intermédiaire d'un joint d'étanchéité (21) sur l'extrémité supérieure (19) de la première partie (2), le fond du réservoir (22) pour le liquide à faire passer à travers la matière, présentant une ouverture de sortie (23) se situant au-dessus du logement (5) destiné à contenir cette matière poreuse.

8. Colonne suivant l'une ou l'autre des revendications 6 et 7, caractérisée en ce que la première partie précitée (2) est constituée d'une cartouche échangeable (16) contenant une matière poreuse (28) pouvant être traversée par un liquide, montée de manière amovible entre ladite troisième partie (17) comprenant le réservoir (6) pour le liquide à faire passer à travers la matière poreuse (28) et la deuxième partie (3) présentant le réservoir de collection (7) pour le liquide ayant traversé la matière poreuse.

9. Dispositif suivant l'une quelconque des revendications 4 à 8, caractérisé en ce qu'au moins une des parties précitées (2, 3, 17) présente au moins un embout conique (10, 18) s'adaptant sur une paroi de conicité sensiblement analogue (9, 27) de l'autre partie située en-dessous de cet embout.

10. Colonne suivant l'une quelconque des revendications 1 à 9, caractérisée en ce qu'au moins la deuxième partie (3), formant la partie inférieure de la colonne (1), présente une forme extérieure compatible avec des compartiments (24) d'une centrifugeuse (25).

11. Colonne suivant la revendication 10, caractérisée en ce que les parties démontables précitées (2, 3, 17) adaptées l'une sur l'autre, forment un ensemble ayant l'allure d'une éprouvette pouvant être placé dans un compartiment correspondant d'une centrifugeuse (25).

0184571

FIG. 1

FIG. 2

FIG. 3

FIG. 4

1
17
2
3

FIG. 7
16

FIG. 8
16

FIG. 9
16a  16b

17

21
19
2
10
11

3
13

FIG. 5

4
17
23  22
18

19
20
21
2
16
5
12
11
10

9
26 7  8
3

FIG. 6

0184571

FIG.3

29

30

28

27

25

20

26

7

33 34

32

35

36 8

Office européen
des brevets

**RAPPORT DE RECHERCHE EUROPEENNE**

EP 85 87 0163

## DOCUMENTS CONSIDERES COMME PERTINENTS

| Categorie | Citation du document avec indication, en cas de besoin, des parties pertinentes | Revendication concernee | CLASSEMENT DE LA DEMANDE (Int Cl 4) |
|---|---|---|---|
| X | EP-A-0 021 065 (ABIMED)<br>* document entier * | 1,2 | B 01 L 3/14<br>G 01 N 33/48 |
| X | DE-A-2 912 675 (E.I. DU PONT DE NEMOURS)<br>* document entier * | 1,2 | |
| A | | 3,4 | |
| X | GB-A-1 548 026 (D.L. BLOXAM)<br>* document entier * | 1,2 | |
| A | | 3,4 | |
| A | US-A-4 092 113 (S.M. HARDY)<br>* document entier * | 1-4 | |

DOMAINES TECHNIQUES
RECHERCHES (Int Cl 4)

G 01 N 1/00
G 01 N 33/00
B 01 L 3/00

Le present rapport de recherche a été établi pour toutes les revendications

| Lieu de la recherche<br>BERLIN | Date d achèvement de la recherche<br>21-02-1986 | Examinateur<br>BRISON O.P. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82